# EUROPEAN PATENT SPECIFICATION

(11) **EP 3 958 890 B1**
(45) Date of publication and mention of the grant of the patent: **20.11.2024**
(21) Application number: 20794291.3
(22) Date of filing: 21.04.2020
(51) Int. Cl.: C12N 9/24, A61K 38/47, C12N 9/26

(54) **BETA-AMYLASE VARIANTS**
BETA-AMYLASE-VARIANTEN
VARIANTS DE BÊTA-AMYLASE

(30) Priority: 23.04.2019 US 201962837328 P
(43) Date of publication of application: 02.03.2022
(73) Proprietor: BASF SE, 67056 Ludwigshafen am Rhein (DE)
(72) Inventor: URBINA, Hugo, San Diego, CA 92121 (US); CHANG, Kristine, W., San Diego, CA 92121 (US); TRAN, Tuan, San Diego, CA 92121 (US); LOWE, Gina Ning, Lee, San Diego, CA 92121 (US); HUSTON DAVENPORT, Adrienne, San Diego, CA 92121 (US); TAN, XUQIU, San Diego, CA 92121 (US); LI, Tong, San Diego, CA 92121 (US); JENEWEIN, Stefan, 67056 Ludwigshafen Am Rhein (DE); SEEMAYER, Stefan, 67056 Ludwigshafen Am Rhein (DE); KUTSCHER, Jochen, 89257 Illertissen (DE)
(74) Representative: Meissner Bolte Partnerschaft mbB
(86) International application number: PCT/US2020/029122
(87) International publication number: WO 2020/219450

(56) References cited:
- WO-A1-2015/021601
- WO-A1-2018/209026
- WO-A1-2021/074056
- WO-A2-2004/081171
- WO-A2-2019/081976
- US-A- 5 082 781
- US-A1- 2008 102 497
- US-A1- 2012 225 164
- KITAMOTO N ET AL: "CLONING AND SEQUENCING OF THE GENE ENCODING THERMOPHILIC BETA-AMYLASE OF CLOSTRIDIUM THERMOSULFUROGENES", JOURNAL OF BACTERIOLOGY, AMERICAN SOCIETY FOR MICROBIOLOGY, US, vol. 170, no. 12, 1 December 1988 (1988-12-01), pages 5848 - 5854, XP000617232, ISSN: 0021-9193

## Description

### FIELD OF THE INVENTION

The present invention relates to variants of a beta-amylase which have an increased % residual exoamylase activity compared to the parent beta-amylase after heat treatment. The present invention also relates to methods of making the variant beta-amylase and the use of the variant beta-amylase in baking, detergents, personal care products, in the processing of textiles, in pulp and paper processing, in the production of ethanol, lignocellulosic ethanol or syrups and as viscosity breaker in oilfield and mining industries.

### BACKGROUND OF THE INVENTION

Bread has been a staple of human nutrition for thousands of years. Bread is usually made by combining a flour, water, salt, yeast, and/or other food additives to make a dough or paste; then the dough is baked to make bread. Enzymes are known to be useful in baking because the enzymes' effects on the baking process may be similar or better than the effects of the chemical alternatives. Several different enzymes may be used for making bread, for example amylase enzymes have been known to help maintain freshness over time (anti-staling or hardness) and maintain resilience overtime. The staling of bread is caused by the crystallization of amylopectin which takes place in starch granules after baking. When bread stales, it loses softness and moisture of the crumbs which become less elastic.

WO2015021601 relates to a process for producing high maltose syrup in which a starch substrate is contacted with a thermostable alpha amylase and a thermoresistant maltose-producing enzyme at a temperature zero to thirty degree above the starch gelatinization temperature. In this context, D2 discloses a *Thermoanaerobacterium thermosulfurigenes* beta-amylase (SEQ ID NO: 3) presenting 100% identity to SEQ ID NO: 1 of the present application.

Hence, there is still a need for an amylase that may provide fresh bread over a longer time than what is currently available or an amylase enzyme that may provide bread that is better than fresh over time.

One solution to this problem are the variant polypeptides having beta-amylase enzyme activity that meet or exceed these industrial requirements. In addition, the beta-amylase variants may be used in animal feed, detergents, personal care products, processing of textiles, pulp and paper processing, in the production of ethanol, in the production lignocellulosic ethanol, in the production of syrups, or as viscosity breakers in oilfield and mining industries.

### SUMMARY OF THE INVENTION

The present inventors have surprisingly found that introducing amino acid modifications in the amino acid sequence of an beta-amylase increases the exoamylase activity of the variant compared to the activity of the parent enzyme.

Accordingly, the present invention relates to a variant polypeptide of the beta-amylase according to SEQ ID No. 1 having beta-amylase activity and comprising an amino acid sequence which is at least 80% identical to the sequence according to SEQ ID No. 1, which amino acid sequence comprises amino acid substitutions D25K, L27C, S220L, A364P, N369P, S398P and at least a first further amino acid modification at an amino acid residue position number 502 in the numbering of SEQ ID No. 1.

In one embodiment, the first further amino acid modification is an amino acid substitution, insertion, deletion, or any combination thereof.

In one embodiment, the first further amino acid modification is an amino acid substitution, and the amino acid substitution is a conservative amino acid substitution.

In one embodiment, the first further amino acid modification is the amino acid substitution T502E, in the numbering of SEQ ID No. 1.

In one embodiment, the first further amino acid modification is a combination of amino acid modifications, and the combination of amino acid modifications is a combination of amino acid substitutions which is selected from the group consisting of:
(h) T494E, T502E; and
(i) N419G, T494E, T502E ;
in the numbering of SEQ ID No. 1.

In one embodiment, the first further amino acid modification is a combination of amino acid modifications, and the combination of amino acid modifications is N419, T494E, T502 in the numbering of SEQ ID No. 1, and the polypeptide comprises at least a second further amino acid modification.

In one embodiment, the second further amino acid modification is an amino acid substitution, insertion, deletion, or any combination thereof.

In one embodiment, the second further amino acid modification is an amino acid substitution, and the amino acid substitution is a conservative amino acid substitution.

In one embodiment, the second further amino acid modification is an amino acid substitution selected from the group consisting of: P13S, A206W, V208C, Y236I, G276D, M318L, C375I, Y435P/E, N438K, and P463T or a combination thereof in the numbering of SEQ ID No. 1.

In one embodiment, the second further one amino acid modification is a combination of amino acid modifications, and the combination of amino acid modifications is a combination of amino acid substitutions which is selected from the group consisting of:
(j) A206W, V208C, G276D, M318L, C375I, Y435P, N438K, P463T;
(k) P13S, V208C, Y236I, G276D, M318L, C375I, Y435P, N438K, P463T;
(l) P13S, A206W, V208C, Y236I, G276D, M318L, C375I, Y435E, N438K, P463T;
(m) V208C, G276D, M318L, C375I, Y435P, N438K, P463T;
(n) V208C, G276D, C375I, Y435P, N438K, P463T;
(o) A206W, V208C, G276D, C375I, Y435E, N438K, P463T;
(p) A206W, V208C, G276D, C375I, Y435P, N438K, P463T;
(q) P13S, A206W, V208C, Y236I, G276D, C375I, Y435P, N438K, P463T;
(r) A206W, V208C, G276D, M318L, Y435E, N438K, P463T;
(s) A206W, V208C, G276D, M318L, C375I, Y435P, P463T;
(t) P13S, A206W, V208C, Y236I, G276D, M318L, C375I, N438K, P463T;
(u) P13S, V208C, Y236I, G276D, M318L, C375I, N438K, P463T;
(v) P13S, A206W, V208C, Y236I, G276D, M318L, C375I, N438K, P463T;
(w) P13S, A206W, V208C, Y236I, G276D, C375I, Y435P, P463T;
(x) V208C, G276D, M318L, Y435P;
(y) P13S, A206W, V208C, Y236I, G276D, M318L, P463T;
(z) A206W, V208C, G276D, M318L, P463T;
(aa) P13S, V208C, Y236I, G276D, N438K, P463T;
(bb) P13S, A206W, V208C, Y236I, G276D, M318L;
(cc) P13S, A206W, V208C, Y236I,G276D;
(dd) G276D, N419G, T494E, T502E;
(ee) C375I, N419G, T494E, T502E;
(ff) P13S, A206W, V208C, Y236I, G276D;
(gg) T90D, V91C, P269S, C375I; and
(hh) T90D, V91C, P269S, G276D, C375I;
in the numbering of SEQ ID No. 1.

In one embodiment, the variant polypeptide has an increased % residual activity after exposure to a temperature of 80 to 95 degrees Celsius compared to the polypeptide of SEQ ID No. 1 or 2 .

In one embodiment, the variant polypeptide has an increased % residual activity after exposure to a temperature of 86 degrees Celsius compared to the polypeptide of SEQ ID No. 1 or 2 .

In one embodiment, the variant polypeptide has an increased % residual activity after exposure to a temperature of 90 degrees Celsius compared to the polypeptide of SEQ ID No. 1, 2, or 3.

In one embodiment, the variant polypeptide has an increased activity at pH 4.5-6 and a temperature of 80-85 degrees Celsius compared to the polypeptide of SEQ ID No. 1 or 2.

In one embodiment, the variant polypeptide has beta-amylase activity and is a fragment of the full length amino acid sequence.

In one embodiment, the variant polypeptide comprises a hybrid of at least one variant polypeptide according to any one of the preceding embodiments, and a second polypeptide having amylase activity, wherein the hybrid has beta-amylase activity.

The present invention further relates to a composition comprising the variant polypeptide according to any one of the preceding embodiments.

In one embodiment, the composition further comprises a second enzyme.

In one embodiment, the second enzyme is selected from the group consisting of: an alpha-amylase, a lipase, a second beta-amylase, a G4-amylase, a xylanase, a protease, a cellulase, a glucoamylase, an oxidoreductase, a phospholipase, and a cyclodextrin glucanotransferase.

The present invention further relates to a method of making a variant polypeptide comprising: providing a template nucleic acid sequence encoding the inventive polypeptide variant, transforming the template nucleic acid sequence into an expression host, cultivating the expression host to produce the variant polypeptide, and purifying the variant polypeptide.

In one embodiment, the expression host is selected from the group consisting of: a bacterial expression system, a yeast expression system, a fungal expression system, and a synthetic expression system.

In one embodiment, the bacterial expression system is selected from an *E. coli*, a *Bacillus*, a *Pseudomonas*, and a *Streptomyces.*

In one embodiment, the yeast expression system is selected from a *Candida*, a *Pichia*, a *Saccharomyces*, a *Schizosaccharomyces.*

In one embodiment, the fungal expression system is selected from a *Penicillium*, an *Aspergillus*, a *Fusarium*, a *Myceliopthora*, a *Rhizomucor,* a *Rhizopus*, a *Thermomyces*, and a *Trichoderma.*

The present invention further relates to a method of preparing a dough or a baked product prepared from the dough, the method comprising adding an inventive variant polypeptide to the dough and eventually baking the dough.

In one embodiment, the baked product produced by the preceding method exhibits lower hardness and greater resilience after 10 days of storage than a baked product produced by an otherwise identical method in which no variant polypeptide is added.

The present invention further relates to a use of the inventive variant polypeptide for processing starch.

The present invention further relates to a use of the inventive variant polypeptide for cleaning or washing textiles, hard surfaces, or dishes.

The present invention further relates to a use of the inventive variant polypeptide for making ethanol.

The present invention further relates to a use of the inventive variant polypeptide for treating an oil well.

The present invention further relates to a use of the inventive variant polypeptide for processing pulp or paper.

The present invention further relates to a use of the inventive variant polypeptide for feeding an animal.

The present invention further relates to a use of the inventive variant polypeptide for making syrup.

### BRIEF DESCRIPTION OF THE DRAWINGS

FIG. 1A, 1B and 1C: Temperature profiles at pH 5.5 for different variant polypeptides as well as intermediate parent polypeptide (SEQ ID No.2). Exoamylase activity was measured by PAHBAH assay as laid out in Example 4.
FIG. 2A, 2B and 2C: pH profiles at 80°C for different variant polypeptides as well as intermediate parent polypeptide (SEQ ID No.2). Exoamylase activity was measured by PAHBAH assay as laid out in Example 5.
FIG. 3: Resilience and hardness in bread with or without variant enzymes or Novamyl 3D control after 10 days measured by Texture Profile Analysis (TPA) as laid out in Example 6.

### DETAILED DESCRIPTION OF THE INVENTION

Although the present invention will be described with respect to particular embodiments, this description is not to be construed in a limiting sense.

Before describing in detail exemplary embodiments of the present invention, definitions important for understanding the present invention are given. Unless stated otherwise or apparent from the nature of the definition, the definitions apply to all methods and uses described herein.

As used in this specification and in the appended claims, the singular forms of "a" and "an" also include the respective plurals unless the context clearly dictates otherwise. In the context of the present invention, the terms "about" and "approximately" denote an interval of accuracy that a person skilled in the art will understand to still ensure the technical effect of the feature in question. The term typically indicates a deviation from the indicated numerical value of ±20 %, preferably ±15 %, more preferably ±10 %, and even more preferably ±5 %.

It is to be understood that the term "comprising" is not limiting. For the purposes of the present invention the term "consisting of" is considered to be a preferred embodiment of the term "comprising". If hereinafter a group is defined to comprise at least a certain number of embodiments, this is meant to also encompass a group which preferably consists of these embodiments only.

Furthermore, the terms "first", "second", "third" or "(a)", "(b)", "(c)", "(d)" etc. and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein. In case the terms "first", "second", "third" or "(a)", "(b)", "(c)", "(d)", "i", "ii" etc. relate to steps of a method or use or assay there is no time or time interval coherence between the steps, i.e. the steps may be carried out simultaneously or there may be time intervals of seconds, minutes, hours, days, weeks, months or even years between such steps, unless otherwise indicated in the application as set forth herein above or below.

It is to be understood that this invention is not limited to the particular methodology, protocols, reagents etc. described herein as these may vary. It is also to be understood that the terminology used herein is for the purpose of describing particular embodiments only, and is not intended to limit the scope of the present invention that will be limited only by the appended claims. Unless defined otherwise, all technical and scientific terms used herein have the same meanings as commonly understood by one of ordinary skill in the art.

As discussed above, the present invention is based on the finding that variants of a beta-amylase have an increased exoamylase activity compared to the parent beta-amylase. In baking applications the exoamylase activity is preferred, as it accomplishes the degradation of starch that leads to an anti-staling effect, but does not negatively affect the quality of the final baked product. In contrast, endoamylase activity can negatively affect the quality of the final baked product, as it leads to an accumulation of branched dextrins which for example lead to the production of a sticky or gummy bread crumb.

A "variant polypeptide" refers to an enzyme that differs from its parent polypeptide in its amino acid sequence. A "variant beta-amylase" refers to a beta-amylase that differs from its parent beta-amylase in its amino acid sequence and has beta-amylase activity. Variant polypeptides are described using the nomenclature and abbreviations for single amino acid molecules according to the recommendations of IUPAC for single letter or three letter amino acid abbreviations.

A "parent" polypeptide amino acid sequence is the starting sequence for introduction of amino acid modifications (e.g. by introducing one or more amino acid substitutions, insertions, deletions, or a combination thereof) to the sequence, resulting in "variants" of the parent polypeptide amino acid sequence. A parent polypeptide includes both a wild-type polypeptide amino acid sequence or a synthetically generated polypeptide amino acid sequence that is used as starting sequence for the introduction of (further) changes. Within the present invention the parent polypeptide is preferably the polypeptide having the amino acid sequence according to SEQ ID No. 1. Alternatively, the parent polypeptide may be a polypeptide comprising an amino acid sequence which is at least 90% identical to the amino acid sequence according to SEQ ID No. 1 and which does not have an amino acid modification at any of the following amino acid residues: 13, 25, 27, 90, 91, 131, 132, 148, 185, 196, 198, 205, 206, 208, 209, 210, 214, 220, 222, 236, 239, 251, 269, 276, 318, 364, 369, 375, 389, 419, 435, 438, 463, 469, 494, 499, 502, and 519 compared to the sequence according to SEQ ID No. 1. The parent polypeptide according to SEQ ID No. 1 is described in KITAMOTO, "Cloning and Sequencing of the Gene Encoding Thermophilic B-amylase of Clostridium Thermosulfurogenes" (1988) J. Bacteriology Vol. 170, p. 5848-5854; NCBI_P19584.1 is AAA23204.1.

An "intermediate parent" polypeptide amino sequence herein designated a polypeptide with the amino acid sequence of SEQ ID No. 2. It differs from SEQ ID No. 1 in that it comprises amino acid substitutions D25K, L27C, S220L, A364P, N369P, S398P.

Beta-amylases (E.C. 3.2.1.2), also known as 1,4-α-D-glucan maltohydrolases, glycogenases, and saccharogen amylases, are enzymes that perform hydrolysis of (1->4)-alpha-D-glucosidic linkages in polysaccharides to remove successive maltose units from the non-reducing ends of the chains. They act on, e.g., starch, glycogen and related polysaccharides as well as oligosaccharides, and produce beta-maltose by an inversion. Beta amylases are widely used in manufacturing caramel, maltose, maltodextrin and brewing beer, alcohol and vinegar fermentation industry.

Beta-amylases are characterized in higher plants and microbial sources, for example beta-amylases from microbial sources include those from *Bacillus acidopullulyticus* (U.S. Patent 4,970,158) and *Bacillus flexus* (Matsunaga, Okada and Yamagat, H. and Tsukagishi, N. 1987, J. Bacteriol. (169) 1564-1570, and U.S. Patent 8,486,682). Other beta-amylases from microbial sources are those from *Clostridium thermosulfurogenes* (Kitaoto, 1988, Kitamoto, N., Yamagata, H., Kato, T., Tsukagoshi, N. and Udaka, S. 1998. J. Bacteriol. (170) 5848-5854), U.S. Patent Application Publication 2012/0225164, WO2015/021600, WO2015/021601, EP0337090A1) and *Thermoanaerobacterium thermosulfurigenes* (JP01218589). Based on the beta-amylase originated from *Clostridium thermosulfurogenes*, acid-resistant beta-amylases were developed (CN103695386 and CN 103881993). In addition, amylase enzymes are disclosed in, e.g., WO2002/068589, WO2002/068597, WO2003/083054, WO2004/042006, WO2008/080093, WO2013/116175, and WO2017/106633.

Commercial amylase enzymes used in food processing and baking include Veron^{®} from AB Enzymes; BakeDream^{®}, BakeZyme^{®}, and Panamore^{®} available from DSM; POWERSoft^{®}, Max-LIFE^{™}, POWERFlex^{®}, and POWERFresh^{®} available from DuPont; and Fungamyl^{®}, Novamyl^{®}, OptiCake^{®}, and Sensea^{®} available from Novozymes.

The beta-amylase activity can be determined by various assays known to the person skilled in the art, including the BCA Reducing Ends Assay (Smith, P.K. (1985) Anal. Biochem. 150 (1): 76-85), the PAHBAH assay (Lever (1972) Anal. Biochem. 47: 273-279), the iodine assay (Fuwa (1954) J. Biochem. 41: 583-603), the Betamyl-3 assay available from Megazyme.

The variant polypeptides of the present invention are characterized in that they have an increased % residual exoamylase activity compared to the parent polypeptide, preferably compared to the polypeptide with the amino acid sequence according to SEQ ID No.1, and compared to the polypeptide with the amino acid sequence according to SEQ ID No. 2. Preferably, the variant polypeptides of the present invention are further characterized in that they have an increased % residual exoamylase activity compared to the polypeptide with the amino acid sequence according to SEQ ID No. 3. The term "exoamylase activity" is intended to mean the cleavage of a starch molecule from the non-reducing end of the substrate as described above. In contrast, "endoamylase activity" means that α-D-(1->4)-O-glucosidic linkages within the starch molecule are cleaved in a random fashion. The term "% residual activity" means the percentage of exoamylase activity that is still exhibited after thermal inactivation of the variant or parent polypeptide for a certain time compared with a reference sample which has not undergone thermal treatment.

% residual exoamylase activity is determined by measuring the activity of a polypeptide (e.g. a variant polypeptide, a parent polypeptide, an intermediate parent polypeptide) before and after heat treatment, for example by PAHBAH assay (see above). The activity observed after heat treatment is then expressed as the percentage of the activity observed before heat treatment.

An increased % residual exoamylase activity means that the % residual activity is higher for a variant polypeptide than for a parent or intermediate parent polypeptide. An example of such a determination is provided in the examples section herein.

"Sequence Identity", "% sequence identity", "% identity", "% identical" or "sequence alignment" means a comparison of a first amino acid sequence to a second amino acid sequence, or a comparison of a first nucleic acid sequence to a second nucleic acid sequence and is calculated as a percentage based on the comparison. The result of this calculation can be described as "percent identical" or "percent ID."

Generally, a sequence alignment can be used to calculate the sequence identity by one of two different approaches. In the first approach, both mismatches at a single position and gaps at a single position are counted as non-identical positions in final sequence identity calculation. In the second approach, mismatches at a single position are counted as non-identical positions in final sequence identity calculation; however, gaps at a single position are not counted (ignored) as non-identical positions in final sequence identity calculation. In other words, in the second approach gaps are ignored in final sequence identity calculation. The difference between these two approaches, i.e. counting gaps as non-identical positions vs ignoring gaps, at a single position can lead to variability in the sequence identity value between two sequences.

A sequence identity is determined by a program, which produces an alignment, and calculates identity counting both mismatches at a single position and gaps at a single position as non-identical positions in final sequence identity calculation. For example program Needle (EMBOS), which has implemented the algorithm of Needleman and Wunsch (Needleman and Wunsch, 1970, J. Mol. Biol. 48: 443-453), and which calculates sequence identity by first producing an alignment between a first sequence and a second sequence, then counting the number of identical positions over the length of the alignment, then dividing the number of identical residues by the length of an alignment, then multiplying this number by 100 to generate the % sequence identity [% sequence identity = (# of Identical residues / length of alignment) x 100)].

A sequence identity can be calculated from a pairwise alignment showing both sequences over the full length, so showing the first sequence and the second sequence in their full length ("Global sequence identity"). For example, program Needle (EMBOSS) produces such alignments; % sequence identity = (# of identical residues / length of alignment) x 100)].

A sequence identity can be calculated from a pairwise alignment showing only a local region of the first sequence or the second sequence ("Local Identity"). For example, program Blast (NCBI) produces such alignments; % sequence identity = (# of Identical residues / length of alignment) x 100)].

A sequence alignment is calculated wherein mismatches at a single position are counted as non-identical positions in final sequence identity calculation; however, gaps at a single position are not counted (ignored) as non-identical positions in final sequence identity calculation. The sequence alignment is generated by using the algorithm of Needleman and Wunsch (J. Mol. Biol. (1979) 48, p. 443-453). Preferably, the program "NEEDLE" (The European Molecular Biology Open Software Suite (EMBOSS)) is used with the programs default parameter (gap open=10.0, gap extend=0.5 and matrix=EBLOSUM62). Then, a sequence identity can be calculated from the alignment showing both sequences over the full length, so showing the first sequence and the second sequence in their full length ("Global sequence identity"). For example: % sequence identity = (# of identical residues / length of alignment) x 100)].

The variant polypeptides are described by reference to an amino acid sequence which is at least n% identical to the amino acid sequence of the respective parent enzyme with "n" being an integer between 80 and 100. The variant polypeptides include enzymes that are at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98% or at least 99% identical when compared to the full length amino acid sequence of the parent beta-amylase according to SEQ ID No. 1, wherein the enzyme variant has beta-amylase activity and an increased % residual exoamylase activity compared to the parent polypeptide according to SEQ ID No. 1 and the polypeptide according to SEQ ID No. 2.

The variant polypeptide comprises amino acid substitutions D25K, L27C, S220L, A364P, N369P, S398P in the numbering of SEQ ID No. 1 and at least a first further amino acid modification at an amino acid residue position number 502 in the numbering of SEQ ID No. 1.. That is, in addition to the amino acid substitutions D25K, L27C, S220L, A364P, N369P, S398P, at least one further amino acid at an amino acid residue position number 502 in the numbering of SEQ ID No. 1.is modified. The term "amino acid modification" means that the amino acid sequence of the variant polypeptide is modified compared to the amino acid sequence of the parent polypeptide, preferably the polypeptide according to SEQ ID No. 1. The term "amino acid modification" is not intended to comprise modifications to an amino acid residue itself, such as, but not limited to, phosphorylation, myristoylation, palmitoylation, isoprenylation, acetylation, alkylation, amidation, gamma-carboxylation or glycoslation. The term "amino acid modification" includes amino acid substitution, amino acid insertion and amino acid deletion. Hence, the variant polypeptide of the present invention comprises at least a first further amino acid substitution, amino acid insertion and/or amino acid deletion compared to the parent polypeptide, preferably the polypeptide according to SEQ ID No. 1. Preferably, the at least first further amino acid modification is at least one amino acid substitution at an amino acid residue position number 502 in the numbering of SEQ ID No. 1.

"Amino acid substitutions" are described by providing the original amino acid residue in the parent polypeptide followed by the number of the position of this amino acid residue within the amino acid sequence. For example, a substitution of amino acid residue 13 means that the amino acid of the parent at position 13 can be substituted with any of the 19 other amino acid residues and is designated as P13. In addition, a substitution can be described by providing the original amino acid residue in the parent polypeptide followed by the number of the position of this amino acid residue within the amino acid sequence and followed by the specific substituted amino acid within the variant polypeptide. For example, the substitution of glycine at position 22 with glutamine is designated as "Pro12Ser" or "P13S". If more than one specific amino acid substitution follows the position number, e.g. "C375I/V", the parent amino acid (here: cysteine) at the indicated position (here: position 375) can be substituted by any one of the listed substituted amino acids (here: either isoleucine or valine). Combinations of substitutions are described by inserting comas between the amino acid residues, for example: G22Q, P35K, S59P, W128Y, D256A; represent a combination of substitutions of five different amino acid residues when compared to a parent polypeptide. Variants having a substitution on the amino acid level are encoded by a nucleic acid sequence which differs from the parent nucleic acid sequence encoding the parent polypeptide at least in the position encoding the substituted amino acid residue.

The amino acid substitution in the variant polypeptide may be a conservative amino acid substitution. A "conservative amino acid substitution" or "substitution with a related amino acid" means replacement of one amino acid residue in an amino acid sequence with a different amino acid residue having a similar property at the same position compared to the parent amino acid sequence. Some examples of a conservative amino acid substitution include, but are not limited to, replacing a positively charged amino acid residue with a different positively charged amino acid residue; replacing a polar amino acid residue with a different polar amino acid residue; replacing a non-polar amino acid residue with a different non-polar amino acid residue, replacing a basic amino acid residue with a different basic amino acid residue, or replacing an aromatic amino acid residue with a different aromatic amino acid residue.

A list of conservative amino acid substitutions is provided in the Table below (see for example Creighton (1984) Proteins. W.H. Freeman and Company (Eds)).

| **Residue** | **Conservative Substitution(s)** | **Residue** | **Conservative Substitution(s)** |
|---|---|---|---|
| Ala | Ser | Leu | Ile, Val |
| Arg | Lys | Lys | Arg, Gln |
| Asn | Gln, His | Met | Leu, Ile |
| Asp | Glu | Phe | Met, Leu, Tyr |
| Gln | Asn | Ser | Thr, Gly |
| Cys | Ser | Thr | Ser, Val |
| Glu | Asp | Trp | Tyr |
| Gly | Pro | Tyr | Trp, Phe |
| His | Asn, Gln | Val | Ile, Leu |
| Ile | Leu, Val | | |

An "amino acid insertion" is described by providing the number of the position within the amino acid sequence behind which the amino acid is inserted followed by an apostrophe and the specific inserted amino acid residue . For example, the insertion of serine behind position 84 is designated as "84'S". Variants having an insertion on the amino acid level are encoded by a nucleic acid sequence which differs from the parent nucleic acid sequence encoding the parent polypeptide at least in the position encoding the inserted amino acid residue.

An "amino acid deletion" is described by providing the number of the position within the amino acid sequence at which the amino acid residue is deleted followed by a delta and the specific deleted amino acid residue . For example, the deletion of glycine on position 10 is designated as "10ΔG". Variants having deletions on the amino acid level are encoded by a nucleic acid sequence which differs from the parent nucleic acid sequence encoding the parent polypeptide at least at the position encoding the deleted amino acid residue.

In one embodiment, the variant polypeptide comprises amino acid substitutions D25K, L27C, S220L, A364P, N369P, S398P and at least a first further amino acid modification at an amino acid residue position number 502 in the numbering of SEQ ID No. 1.

The first further amino acid modification is an amino acid substitution, insertion, deletion, or any combination thereof. For example, the first further amino acid modification is an amino acid substitution, and the amino acid substitution is a conservative amino acid substitution. Preferably, the first further amino acid modification is an amino acid substitution T502E in the numbering of SEQ ID No. 1.

In a more preferred embodiment, the first further amino acid modification is a combination of amino acid modifications, and the combination of amino acid modifications is a combination of amino acid substitutions which is selected from the group consisting of:
(a) T494E, T502E; and
(b) N419G, T494E, T502E;
in the numbering of SEQ ID No. 1.

The above variant polypeptides are characterized in that they have an increased % residual activity after exposure to a temperature of 80 to 95, e.g. degrees Celsius, e.g. 80°C, 81°C, 82°C, 83°C, 84°C, 85°C, 86°C, 87°C, 88°C, 89°C, 90°C, 91°C 92°C, 93°C, 94°C, 95°C, compared to the polypeptide of SEQ ID No. 1 or 2. Preferably, the above variant polypeptides have an increased % residual activity after exposure to a temperature of 86 degrees Celsius compared to the polypeptide of SEQ ID No. 1 or 2.

In a particularly preferred embodiment, the first further amino acid modification is a combination of amino acid modifications, the combination of amino acid modifications is N419, T494E, T502 in the numbering of SEQ ID No. 1, and the polypeptide comprises at least a second further amino acid modification. That is, in addition to amino acid substitutions D25K, L27C, S220L, A364P, N369P, S398P, 419, T494E, and T502 (as shown in SEQ ID No. 3), at least one other amino acid at a different position is modified.

In an embodiment, the second further amino acid modification is an amino acid substitution, insertion, deletion, or any combination thereof. For example, the second further amino acid modification is an amino acid substitution, and wherein the amino acid substitution is a conservative amino acid substitution. Preferably, the second further amino acid modification is an amino acid substitution selected from the group consisting of: P13S, A206W, V208C, Y236I, G276D, M318L, C375I, Y435P/E, N438K, and P463T or a combination thereof in the numbering of SEQ ID No. 1.

In a particularly preferred embodiment, the second further one amino acid modification is a combination of amino acid modifications, and the combination of amino acid modifications is a combination of amino acid substitutions which is selected from the group consisting of:
(c) A206W, V208C, G276D, M318L, C375I, Y435P, N438K, P463T;
(d) P13S, V208C, Y236I, G276D, M318L, C375I, Y435P, N438K, P463T;
(e) P13S, A206W, V208C, Y236I, G276D, M318L, C375I, Y435E, N438K, P463T;
(f) V208C, G276D, M318L, C375I, Y435P, N438K, P463T;
(g) V208C, G276D, C375I, Y435P, N438K, P463T;
(h) A206W, V208C, G276D, C375I, Y435E, N438K, P463T;
(i) A206W, V208C, G276D, C375I, Y435P, N438K, P463T;
(j) P13S, A206W, V208C, Y236I, G276D, C375I, Y435P, N438K, P463T;
(k) A206W, V208C, G276D, M318L, Y435E, N438K, P463T;
(l) A206W, V208C, G276D, M318L, C375I, Y435P, P463T;
(m) P13S, A206W, V208C, Y236I, G276D, M318L, C375I, N438K, P463T;
(n) P13S, V208C, Y236I, G276D, M318L, C375I, N438K, P463T;
(o) P13S, A206W, V208C, Y236I, G276D, M318L, C375I, N438K, P463T;
(p) P13S, A206W, V208C, Y236I, G276D, C375I, Y435P, P463T;
(q) V208C, G276D, M318L, Y435P;
(r) P13S, A206W, V208C, Y236I, G276D, M318L, P463T;
(s) A206W, V208C, G276D, M318L, P463T;
(t) P13S, V208C, Y236I, G276D, N438K, P463T;
(u) P13S, A206W, V208C, Y236I, G276D, M318L;
(v) P13S, A206W, V208C, Y236I,G276D;
(w) G276D, N419G, T494E, T502E ;
(x) C375I, N419G, T494E, T502E ;
(y) P13S, A206W, V208C, Y236I, G276D;
(z) T90D, V91C, P269S, C375I; and
(aa) T90D, V91C, P269S, G276D, C375I;
in the numbering of SEQ ID No. 1.

Preferably, the variant polypeptides comprising a second further amino acid modification have an increased % residual activity after exposure to a temperature of 90 degrees Celsius compared to the polypeptide of SEQ ID No. 1, 2, or 3.

Preferably, the variant polypeptides comprising a second further amino acid modification have an increased activity at pH 4.5-6 and a temperature of 80-85 degrees Celsius, e.g. 80°C, 81°C, 82°C, 83°C, 84°C, or 85°C, compared to the polypeptide of SEQ ID No. 1 or 2.

The variant polypeptide may be a fragment. A "fragment" of a beta-amylase is understood to refer to a smaller part of the beta-amylase which consists of a contiguous amino acid sequence found in the amino acid sequence of the beta-amylase and which has beta-amylase activity. The skilled person knows that for a fragment to be enzymatically active the fragment has to comprise at least the amino acids present in the catalytic center of the beta-amylase. These amino acids are either known for a given beta-amylase or can easily be identified by the skilled person, for example by homology screening or mutagenesis. Further the fragment must comprise the indicated modified residues. Preferably, the fragment of the beta-amylase has an increased % residual exoamylase activity compared to the full-length polypeptide according to SEQ ID No. 1. Preferably, the fragment comprises at least 70%, at least 80 %, at least 85%, at least 90 %, at least 95%, at least 96%, at least 97%, at least 98 %, or at least 99% of the amino acids of the full-length polypeptide according to SEQ ID No.1.

The variant polypeptide may comprise a hybrid of at least one variant polypeptide and a second polypeptide having amylase activity, wherein the hybrid has beta-amylase activity. For example, the variant polypeptide having beta-amylase activity may be a hybrid of more than one beta-amylase enzyme. A "hybrid" or "chimeric" or "fusion protein" means that a domain of a first variant polypeptide beta-amylase is combined with a domain of a second beta-amylase to form a hybrid amylase and the hybrid has beta-amylase activity. Preferably, the hybrid beta-amylase has an increased % residual exoamylase activity compared to the polypeptide according to SEQ ID No. 1. A domain of variant polypeptides having beta-amylase enzyme activity can be combined with a domain of a commercially available amylase, such as Veron^{®} available from AB Enzymes; BakeDream^{®}, BakeZyme^{®}, and Panamore^{®} available from DSM; POWERSoft^{®}, Max-LIFE^{™}, POWERFlex^{®}, and POWERFresh^{®} available from DuPont; and Fungamyl^{®}, Novamyl^{®}, OptiCake^{®}, and Sensea^{®} available from Novozymes. In addition, domains from various amylase enzymes can be recombined into a single enzyme, wherein the enzyme has beta-amylase activity. Preferably, the hybrid beta-amylase comprising domains from various amylase enzymes has an increased % residual exoamylase activity compared to the polypeptide according to SEQ ID No. 1.

The variant polypeptides having beta-amylase activity may be a "mature polypeptide." A mature polypeptide means an enzyme in its final form including any post-translational modifications, glycosylation, phosphorylation, truncation, N-terminal modifications, C-terminal modifications or signal sequence deletions. A mature polypeptide can vary depending upon the expression system, vector, promoter, and/or production process.

"Enzymatic activity" means at least one catalytic effect exerted by an enzyme. Enzymatic activity is expressed as units per milligram of enzyme (specific activity) or molecules of substrate transformed per minute per molecule of enzyme (molecular activity). Enzymatic activity can be specified by the enzymes actual function and within the present invention means beta-amylase activity as described above.

Enzymatic activity changes during storage or operational use of the enzyme. The term "enzyme stability" relates to the retention of enzymatic activity as a function of time during storage or operation.

To determine and quantify changes in catalytic activity of enzymes stored or used under certain conditions over time, the "initial enzymatic activity" is measured under defined conditions at time zero (100%) and at a certain point in time later (x%). By comparison of the values measured, a potential loss of enzymatic activity can be determined in its extent. The extent of enzymatic activity loss determines the stability or non-stability of an enzyme.

Parameters influencing the enzymatic activity of an enzyme and/or storage stability and/or operational stability are for example pH, temperature, and presence of oxidative substances.

"pH stability", refers to the ability of a protein to function over a specific pH range. In general, most enzymes are working under conditions with rather high or rather low pH ranges.

The variant polypeptide may be active over a broad pH at any single point within the range from about pH 4.0 to about pH 12.0. The variant polypeptide having beta-amylase activity is active over a range of pH 4.0 to pH 11.0, pH 4.0 to pH 10.0, pH 4.0 to pH 9.0, pH 4.0 to pH 8.0, pH 4.0 to pH 7.0, pH 4.0 to pH 6.0, or pH 4.0 to pH 5.0. The variant polypeptide having beta-amylase enzyme activity is active at pH 4.0, pH 4.1, pH 4.2, pH 4.3, pH 4.4, pH 4.5, pH 4.6, pH 4.7, pH 4.8, pH 4.9, pH 5.0, pH 5.1, pH 5.2, pH 5.3, pH 5.4, pH 5.5, pH 5.6, pH 5.7, pH 5.8, pH 5.9, pH 6.0, pH 6.1, pH 6.2, pH 6.3, pH 6.4, pH 6.5, pH 6.6, pH 6.7, pH 6.8, pH 6.9, pH 7.0, pH 7.1, pH 7.2, pH 7.3, pH 7.4, pH 7.5, pH 7.6, pH 7.7, pH 7.8, pH 7.9, pH 8.0, pH 8.1, pH 8.2, pH 8.3, pH 8.4, pH 8.5, pH 8.6 pH 8.7, pH 8.8 pH 8.9, pH 9.0, pH 9.1, pH 9.2, pH 9.3, pH 9.4, pH 9.5, pH 9.6, pH 9.7, pH 9.8, pH 9.9, pH 10.0, pH 10.1, pH 10.2, pH 10.3, pH 10.4, pH 10.5, pH 10.6, pH 10.7, pH 10.8, pH 10.9, pH 11.0, pH 11.1, pH 11.2, pH 11.3, pH 11.4, pH 11.5, pH 11.6, pH 11.7, pH 11.8, pH 11.9, pH 12.0, pH 12.1, pH 12.2, pH 12.3, pH 12.4, and pH 12.5, pH 12.6, pH 12.7, pH 12.8, pH 12.9, and higher.

The terms "thermal stability" and "thermostability" refer to the ability of a protein to function over a temperature range. In general, most enzymes have a finite range of temperatures at which they function. In addition to enzymes that work at mid-range temperatures (e.g., room temperature), there are enzymes that are capable of working at very high or very low temperatures.

Thermostability is characterized by what is known as the Tso value (also called half-life). The T₅₀ indicates the temperature at which 50% residual activity is still present after thermal inactivation for a certain time compared with a sample which has not undergone thermal treatment.

The terms "thermal tolerance" and "thermotolerance" refer to the ability of a protein to function after exposure to a specific temperature, such as a very high or very low temperature. A thermotolerant protein may not function at the exposure temperature, but will function once returned to a favorable temperature, i.e. exhibit a high % residual activity. This is especially important in baking, where very high temperatures must be endured by enzymes in the dough during the baking process. A beta amylase added to the dough must thus be able to endure high baking temperatures, and, after cooling of the baking product, still exhibit activity at the lower temperatures to prevent staling.

Variant polypeptides may be active over a broad temperature used at any time during a baking process, wherein the temperature is any point in the range from about 20°C to about 60°C. The variant polypeptides having beta-amylase enzyme activity are active at a temperature range from 20°C to 55°C, 20°C to 50°C, 20°C to 45°C, 20°C to 40°C, 20°C to 35°C, 20°C to 30°C, or 20°C to 25°C. Preferably, the variant polypeptides having beta-amylase enzyme activity are active at a temperature of at least 19°C, 20°C, 21°C, 22°C, 23°C, 24°C, 25°C, 26°C, 27°C, 28°C, 29°C, 30°C, 31°C, 32°C, 33°C, 34°C, 35°C, 36°C, 37°C, 38°C, 39°C, 40°C, 41°C, 42°C, 43°C, 44°C, 45°C, 46°C, 47°C, 48°C, 49°C, 50°C, 51°C, 52°C, 53°C, 54°C, 55°C, 56°C, 57°C, 58°C, 59°C, 60°C, 61°C, 62°C, 63°C, 64°C, 65°C, 66°C, 67°C, 68°C, 69°C, 70°C, 71°C, 72°C, 73°C, 74°C, 75°C, 76°C, 77°C, 78°C, 79°C, 80°C, 81°C, 82°C, 83°C, 84°C, 85°C, 86°C, 87°C, 88°C, 89°C, 90°C, or higher temperatures.

The variant polypeptides having beta-amylase enzyme activity may be used or formulated alone or as a mixture of enzymes.

The formulation containing the variant polypeptide of the present invention may be a solid form such as powder, a lyophilized preparation, a granule, a tablet, a bar, a crystal, a capsule, a pill, a pellet, or in a liquid form such as in an aqueous solution, an aerosol, a gel, a paste, a slurry, an aqueous/oil emulsion, a cream, a capsule, or in a vesicular or micellar suspension.

The variant polypeptide of the present invention may be used in combination with at least one other enzyme. The other enzyme may be from the same class of enzymes, for example, may be a second beta-amylase. The other enzyme may also be from a different class of enzymes, for example may be a lipase. The combination with at least one other enzyme may be a composition comprising at least three enzymes. The three enzymes may be from the same class of enzymes, for example the combination may comprise the variant polypeptide of the present invention, a second amylase, and a third amylase; or the enzymes may be from different class of enzymes for example the combination may comprise the variant polypeptide of the present invention, a lipase, and a xylanase.

The second enzyme may be selected from the group consisting of: a second beta-amylase, an alpha-amylase, a glucan 1, 4-alpha-maltotetraohydrolase, also known as exo-maltotetraohydrolase, G4-amylase; a glucan 1,4-alpha-maltohydrolase, also known as maltogenic alpha-amylase, a cyclodextrin glucanotransferase, a glucoamylase; an endo-1,4-beta-xylanase; a xylanase, a cellulase, an oxidoreductase; a phospholipase A1; a phospholipase A2; a phospholipase C; a phospholipase D; a galactolipase, a triacylglycerol lipase, an arabinofuranosidase, a transglutaminase, a pectinase, a pectate lyase, a protease, or any combination thereof. The enzyme combination may comprise the variant polypeptide of the present invention and a lipase, or the enzyme combination may comprise the variant polypeptide of the present invention, a lipase, and a xylanase.

The present invention is also directed to a composition comprising the variant polypeptide of the present invention.

The composition comprising the variant polypeptide of the present invention may also comprise a second enzyme.

Preferably the second enzyme is selected from the group consisting of: a second bet-amylase, a lipase, an alpha-amylase, a G4-amylase, a xylanase, a protease, a cellulase, a glucoamylase, an oxidoreductase, a phospholipase, and a cyclodextrin glucanotransferase.

The composition of the present invention may be used in the preparation of bakery products.

In an aspect, the present invention provides a method of making a variant polypeptide comprising: providing a template nucleic acid sequence encoding the polypeptide variant, transforming the template nucleic acid sequence into an expression host, cultivating the expression host to produce the variant polypeptide, and purifying the variant polypeptide.

Preferably, the variant beta-amylase according to the present invention is a recombinant protein which is produced using bacterial, fungal, yeast, or synthetic expression systems. "Expression system" also means a host microorganism, expression hosts, host cell, production organism, or production strain and each of these terms can be used interchangeably. Examples of expression systems include, but are not limited to: *Aspergillus niger*, *Aspergillus oryzae*, *Hansenula polymorpha*, *Thermomyces lanuginosus*, *Fusarium oxysporum*, *Fusarium heterosporum*, *Escherichia coli*, *Bacillus*, preferably *Bacillus subtilis* or *Bacillus licheniformis*, *Pseudomonas*, preferably *Pseudomonas fluorescens*, *Pichia pastoris* (also known as *Komagataella phaffii*), *Myceliopthora thermophila* (C1), *Schizosaccharomyces pombe*, *Trichoderma*, preferably *Trichoderma reesei* and *Saccharomyces,* preferably *Saccharomyces cerevisiae.*

"Transforming" means the introduction of exogenous DNA into an expression host by methods well known to the person skilled in the art.

"Purifying" means the removal of other cellular material of the expression host from the variant polypeptide by methods well established in the art.

The present invention is also directed to a method of preparing a dough, the method comprising adding the variant polypeptide of the present invention to the dough.

"Dough" is defined as a mixture of flour, salt, yeast and water, which may be kneaded, molded, shaped or rolled prior to baking. In addition, also other ingredients such as sugar, margarine, egg, milk, etc. might be used. The term includes doughs used for the preparation of baked goods, such as bread, rolls, sandwich bread, baguette, ciabatta, croissants, sweet yeast doughs, etc.

The present invention is also directed to a method of preparing a baked product prepared from a dough, the method comprising adding the variant polypeptide of the present invention to the dough and baking the dough, thereby preparing the baked product.

The term "baked products" includes, but is not limited to, baked products such as bread, crispy rolls, sandwich bread, buns, baguette, ciabatta, croissants, noodles, as well as fine bakery wares like donuts, brioche, stollen, cakes, muffins, etc.

Baked products include, but are not limited to: bread, rolls, buns, pastries, cakes, flatbreads, pizza bread, pita bread, wafers, pie crusts, naan, lavish, pitta, focaccia, sourdoughs, noodles, cookies, doughnuts, deep-fried tortillas, pancakes, crepes, croutons, and biscuits. The baked product could also be an edible container such as a cup or a cone.

Baking bread generally involves mixing ingredients to form a dough, kneading, rising, shaping, baking, cooling and storage. The ingredients used for making the dough generally include flour, water, salt, yeast, and other food additives. In the method of the present invention the variant polypeptide of the present invention is one of the ingredients used for making the dough.

Flour is generally made from wheat and may be milled for different purposes such as making bread, pastries, cakes, biscuits pasta, and noodles. Alternatives to wheat flour include, but are not limited to: almond flour, coconut flour, chia flour, corn flour, barley flour, spelt flour, soya flour, hemp flour, potato flour, quinoa, teff flour, rye flour, amaranth flour, arrowroot flour, chick pea (garbanzo) flour, cashew flour, flax meal, macadamia flour, millet flour, sorghum flour, rice flour, tapioca flour, and any combination thereof. Flour type is known to vary between different regions and different countries around the world.

Treatment of flour or dough may include adding inorganic substances, organic substances such as fatty acids, carbohydrates, amino acids, proteins, and nuts. The flour or dough may be pretreated prior to baking by cooling, heating, irradiation, agglomeration, or freeze-drying. In addition, the flour or dough may be pretreated prior to baking by adding enzymes such as the variant polypeptide of the present invention, or micro-organisms, such as yeasts.

Yeast breaks down sugars into carbon dioxide and water. A variety of Baker's yeast, which are usually derived from Saccharomyces cerevisiae, are known to those skilled in the art including, but not limited to: cream yeast, compressed yeast, cake yeast, active dry yeast, instant yeast, osmotolerant yeasts, rapid-rise yeast, deactivated yeast. Other kinds of yeast include nutritional yeast, brewer's yeast, distiller's and wine yeast.

Sweeteners which can be added to the dough include, but are not limited to: liquid sugar, syrups, white (granulated) sugars, brown (raw) sugars, honey, fructose, dextrose, glucose, high fructose corn syrup, molasses, stevia and artificial sweeteners.

Emulsifiers which can be added to the dough include, but are not limited to, diacetyl tartaric acid esters of monoglycerides (DATEM), sodium stearoyl lactylate (SSL), calcium stearoyl lactylate (CSL), ethoxylated mono- and diglycerides (EMG), polysorbates (PS), and succinylated monoglycerides (SMG).

Other food additives which may be used in the methods of baking include: lipids, oils, butter, margarine, shortening, butterfat, glycerol, eggs, diary, non-diary alternatives, thickeners, preservatives, colorants, and enzymes.

Ingredients or additives for baking may be added individually to the dough during the baking process. The ingredients or additives may also be combined with more than one ingredient or additive to form pre-mixes and then the pre-mixes are added to the dough during the baking process. The flour or dough mixtures may be prepared prior to baking including ready-for oven doughs, packaged doughs or packaged batters.

Bakery products may be modified to meet special dietary requirements such as sugar-free diet, gluten-free diet, low fat diet, or any combination thereof. The enzymes may extend shelf-life of a dough-based product or provide antimicrobial (mold-free) effects.

"Bread volume" is the volume of a baked good determined by using a laser scanner (e.g. Volscan Profiler ex Micro Stable System) to measure the volume as well as the specific volume. The term also includes the volume which is determined by measuring the length, the width and the height of certain baked goods.

The use of the variant polypeptide of the present invention in a method of making a dough increases the resilience of the baked product prepared from the dough. The baked product may be stored for five days, 10 days, 15 days or 20 days, before resilience is determined. The resilience can be determined by a texture analyzer test using the Texture Profile Analysis (TPA). The TPA is a two cycle compression test and the resilience is calculated by Recoverable work done divided by hardness work done by the texture analyzer. The resilience of a baked product prepared from dough using the variant polypeptide of the present invention is increased by at least 5% or 8%, preferably by at least 10% or 12%, more preferably by at least 15% or 20% and most preferably by at least 25% or 30%.

The use of the variant polypeptide of the present invention in a method of making a dough decreases the hardness of the baked product prepared from the dough after storage. Typically, the baked product is stored for 10 days, 15 days or 20 days at room temperature, before the hardness is determined. The hardness may be determined according to the AACC 74-09 test, for example using a 35 mm sample and 5 kg load cell. The following parameters may be used in the test: Pre-test speed: 1 mm/sec, Test speed: 5 mm/sec, Post-Test speed: 5 mm/sec, Target Mode: Distance, Distance: 10 mm, Time 5 sec, Trigger Type: Auto (Force), Trigger Force: 5 g. The hardness of a baked product prepared from dough using the variant polypeptide of the present invention is decreased by at least 5% or 8%, preferably by at least 10% or 12%, more preferably by at least 15% or 20%, still more preferably by at least 25% or 30%, and most preferably by at least 35 or 40%.

In a preferred embodiment, the baked product produced by the method of preparing a baked product from a dough exhibits lower hardness and greater resilience after 10 days of storage than a baked product produced in an identical fashion in which no variant polypeptide is added to the dough.

The variant polypeptide of the present invention may be useful for other industrial applications. The variant polypeptide having beta-amylase enzyme activity may be used in a detergent, a personal care product, in the processing of textiles, in pulp and paper processing, in the production of ethanol, lignocellulosic ethanol, or syrups; or as viscosity breakers in oilfield and mining industries.

The following examples are provided for illustrative purposes. It is thus understood that the examples are not to be construed as limiting. The skilled person will clearly be able to envisage further modifications of the principles laid out herein.

### EXAMPLES

### Example 1: General methods

### 1. PAHBAH assay

Quantitation of starch hydrolysis for the beta-amylase and variant enzymes was measured using the 4-Hydroxybenzhydrazide method as described in Lever M. (1972) Anal. Biochem. 47, 273-279, with the following modifications. 112uL of 1% potato amylopectin was reacted with 12.5uL of diluted enzyme at 65°C and samples taken at 60 minutes. The reaction was then quenched by mixing into 100ul 1% PAHBAH reagent. The reaction was heated to 95°C for 6 minutes, cooled to room temperature, and the solution absorption was read at 410nm in a BioTek plate reader.

### 2. Residual Activity

Residual activity was calculated by comparing the activity of each enzyme as measured using the PAHBAH assay before and after a heat challenge. After heating the sample for 10 or 15 minutes, the sample was cooled to room temperature or for 10 minutes to 4°C before being tested, using the PAHBAH assay, at 65°C.

### Example 2: Generation of variant beta-amylase enzymes

The parent enzyme according to SEQ ID No. 1 is described in KITAMOTO, "Cloning and Sequencing of the Gene Encoding Thermophilic B-amylase of Clostridium Thermosulfurogenes" (1988) J. Bacteriology Vol. 170, p. 5848-5854; NCBI_P19584.1 is AAA23204.1. An enzyme according to SEQ ID No. 2, corresponding to SEQ ID No. 1 containing amino acid substitutions D25K, L27C, S220L, A364P, N369P, and S398P, which exhibited improved beta-amylase activity compared to the beta amylase of SEQ ID No. 1 was designed. The enzyme according to SEQ ID No. 2, which will be designated "intermediate parent" herein, was engineered in the lab to generate non-naturally occurring beta-amylase variant enzymes having an increased % residual exoamylase activity after compared to the intermediate parent enzyme. The variant polypeptide enzymes were created starting with the intermediate parent enzyme and evolving it using Gene Site Saturation Mutagenesis (GSSM) as described in at least US 6,562,594, US 6,171,820, and US 6,764,835; Error Prone PCR; and/or Tailored Multi-Site-Combinatorial Assembly (TMSCA), as described in U.S. Patent 9,476,078.

The following variant polypeptides having one amino acid substitution compared to the intermediate parent polypeptide according to SEQ ID No. 2 were generated and % residual activity after a heat challenge as indicated was determined as described in Example 1:

**Table 1: Single GSSM mutations**

| Sample Name | Further Mutation in SEQ ID No.2 | % residual activity after heat challenge (86 °C, 10 min) | % residual activity after heat challenge (84 °C, 10 min, pH 5.5) | % residual activity after heat challenge (86 °C, 10 min; 4 °C, 10 min) |
|---|---|---|---|---|
| BAV01 | N196I | 63 | | |
| BAV02 | M198I | 37 | | |
| BAV03 | I205K | 45 | | |
| BAV04 | A206G | 42 | | |
| BAV05 | A206M | 45 | | |
| BAV06 | A206N | 45 | | |
| BAV07 | A206W | 43 | | |
| BAV08 | N209D | 36 | | |
| BAV09 | S210V | 41 | | |
| BAV10 | T214H | 23 | | |
| BAV11 | I222C | 38 | | |
| BAV12 | V251S | 42 | | |
| BAV13 | V251T | 44 | | |
| BAV14 | P269S | 23 | | |
| BAV15 | P269G | 23 | | |
| BAV16 | M318L | | | 35 |
| BAV17 | C375I | | | 45 |
| BAV18 | C375V | | | 35 |
| BAV19 | N419E | | 68 | |
| BAV20 | N419G | | 84 | |
| BAV21 | N419D | | 79 | |
| BAV22 | N419C | | 68 | |
| BAV23 | Y435E | 41 | | |
| BAV24 | Y435P | 41 | | |
| BAV25 | Y435P | 41 | | |
| BAV26 | N438K | 43 | | |
| BAV27 | N438M | 45 | | |
| BAV28 | N438Q | 42 | | |
| BAV29 | N438Y | 41 | | |
| BAV30 | P463D | 38 | | |
| BAV31 | P463E | 41 | | |
| BAV32 | P463I | 39 | | |
| BAV33 | P463K | 35 | | |
| BAV34 | P463Q | 48 | | |
| BAV35 | P463T | 49 | | |
| BAV36 | P463T | 35 | | |
| BAV37 | P463V | 37 | | |
| BAV38 | P463Y | 38 | | |
| BAV39 | T469V | 33 | | |
| Intermediate parent (SEQ ID No. 2) | | 22 | 60 | 31 |

The following variant polypeptides having a combination of amino acid substitutions compared to the intermediate parent polypeptide according to SEQ ID No. 2 were generated and % residual activity after heat challenge as indicated was determined as described in Example 1:

**Table 2: Multiple GSSM mutations**

| Sample Name | Further Mutation in SEQ ID No.2 | % residual activity after heat challenge (86 °C, 10 min) | % residual activity after heat challenge (84 °C, 10 min, pH 5.5) | % residual activity after heat challenge (86 °C, 10 min; 4 °C, 10 min) |
|---|---|---|---|---|
| BAV40 | K148E-N438A | 38 | | |
| BAV41 | P13S-Y236I | 23 | | |
| BAV42 | T239S-N519D | 23 | | |
| BAV43 | T469I-S499P | 37 | | |
| BAV44 | V208C-G276D | 55 | | |
| BAV45 | L132E+Q131K | 23 | | |
| BAV46 | T90D+V91C | | | 36 |
| BAV47 | T494E-T502E | | 75 | 32 |
| Intermediate parent (SEQ ID No. 2) | | 22 | 60 | 31 |

By enzyme evolution, as described in at least US 6,562,594, US 6,171,820, and US 6,764,835; Error Prone PCR; and/or Tailored Multi-Site-Combinatorial Assembly (TMSCA), as described in U.S. Patent 9,476,078, combinations of the above mutations obtained by GSSM were created in the intermediate parent polypeptide (SEQ ID No. 2), and % residual activity after heat challenge as indicated was determined as described in Example 1:

**Table 3: First round enzyme evolution**

| Sample Name | Further Mutation in SEQ ID No.2 | % residual activity after heat challenge (86 °C, 10 min; 4 °C, 10 min) |
|---|---|---|
| BAV48 (SEQ ID No.3) | N419G-T494E-T502E | 36 |
| Intermediate parent (SEQ ID No. 2) | | 31 |

By enzyme evolution, as described in at least US 6,562,594, US 6,171,820, and US 6,764,835; Error Prone PCR; and/or Tailored Multi-Site-Combinatorial Assembly (TMSCA), as described in U.S. Patent 9,476,078, various of the above mutations obtained by GSSM were combined with those of BAV48 (SEQ ID No.3) in the intermediate parent polypeptide (SEQ ID No.2), and % residual activity after heat challenge as indicated was determined as described in Example 1:

**Table 4: Second round enzyme evolution**

| Sample Name | Further Mutation in SEQ ID No.2 | % residual activity after heat challenge (90 °C, 10 min) | % residual activity after heat challenge (88 °C, 15 min) |
|---|---|---|---|
| BAV 49 | V208C-G276D-N419G-T494E-T502E | | 49 |
| BAV 50 | C375I-N419G-T494E-T502E | | 58 |
| BAV51 | A206W-V208C-G276D-M318L-C375I-Y435P-N438K-P463T-N419G-T494E-T502E | 45 | |
| BAV52 | P13S-V208C-Y236I-G276D-M318L-C375I-Y435P-N438K-P463T-N419G-T494E-T502E | 48 | |
| BAV53 | P13S-A206W-V208C-Y236I-G276D-M318L-C375I-Y435E-N438K-P463T-N419G-T494E-T502E | 45 | |
| BAV54 | V208C-G276D-M318L-C375I-Y435P-N438K-P463T-N419G-T494E-T502E | 45 | |
| BAV55 | V208C-G276D-C375I-Y435P-N438K-P463T-N419G-T494E-T502E | 43 | |
| BAV56 | A206W-V208C-G276D-C375I-Y435E-N438K-P463T-N419G-T494E-T502E | 43 | |
| BAV57 | A206W-V208C-G276D-C375I-Y435P-N438K-P463T-N419G-T494E-T502E | 43 | |
| BAV58 | P13S-A206W-V208C-Y236I-G276D-C375I-Y435P-N438K-P463T-N419G-T494E-T502E | 43 | |
| BAV59 | A206W-V208C-G276D-M318L-Y435E-N438K-P463T-N419G-T494E-T502E | 38 | |
| BAV60 | A206W-V208C-G276D-M318L-C375I-Y435P-P463T-N419G-T494E-T502E | 33 | |
| BAV61 | P13S-A206W-V208C-Y236I-G276D-M318L-C375I-N438K-P463T-N419G-T494E-T502E | 33 | |
| BAV62 | P13S-V208C-Y236I-G276D-M318L-C375IN438K-P463T-N419G-T494E-T502E | 31 | |
| BAV63 | P13S-A206W-V208C-Y236I-G276D-M318L-C375I-N438K-P463T-N419G-T494E-T502E | 31 | |
| BAV64 | P13S-A206W-V208C-Y236I-G276D-C375I-Y435P-P463T-N419G-T494E-T502E | 28 | |
| BAV65 | V208C-G276D-M318L-Y435P-N419G-T494E-T502E | 27 | |
| BAV66 | P13S-A206W-V208C-Y236I-G276D-M318L-P463T-N419G-T494E-T502E | 26 | |
| BAV67 | A206W-V208C-G276D-M318L-P463T-N419G-T494E-T502E | 25 | |
| BAV68 | P13S-V208C-Y236I-G276D-N438K-P463T-N419G-T494E-T502E | 25 | |
| BAV69 | V208C-G276D-P13S-Y236I-A206W-M318L-N419G-T494E-T502E | 23 | |
| BAV70 | V208C-G276D-P13S-Y236I-A206W-N419G-T494E-T502E | 21 | |
| BAV71 | T90D-V91C-P269S-C375IN419G-T494E-T502E | 10 | |
| BAV72 | T90D-V91C-P269S-G276D-C375I-N419G-T494E-T502E | 25 | |
| BAV73 | T90D-V91C-A206W-C208V-P269S-G276D-M318L-C375I-Y435P-N438K-P463T-N419G-T494E-T502E | 47 | |
| BAV48 (SEQ ID No. 3) | N419G-T494E-T502E | 0 | |
| Intermediate parent (SEQ ID No. 2) | | 0 | 0 |

### Example 3: Expression of variant beta-amylases

The variant polypeptides having beta-amylase activity were obtained by constructing expression plasmids containing the encoding polynucleotide sequences, transforming said plasmids into *Pichia pastoris* (*Komagataella phaffii*) and growing the resulting expression strains in the following way.

Fresh *Pichia pastoris* cells of the expression strains were obtained by spreading the glycerol stocks of sequence-confirmed strains onto Yeast extract Peptone Dextrose (YPD) agar plates containing Zeocin. After 2 days, starter seed cultures of the production strains were inoculated into 100 mL of Buffered Glycerol complex Medium (BMGY) using cells from these plates, and grown for 20-24 hours at 30°C and 225-250 rpm. Seed cultures were scaled up by transferring suitable amounts into 2-4 L of BMMY medium in a baffled Fermenter. Fermentations were carried out at 30°C and under 1100 rpm of agitation, supplied via flat-blade impellers, for 48-72 hours. After the initial batch-phase of fermentation, sterile-filtered methanol was added as feed whenever the dissolved oxygen level in the culture dipped below 30%. Alternatively, feed was added every 3 hours at 0.5% v/v of the starting batch culture. The final fermentation broth was centrifuged at 7000xg for 30 mins at 4°C to obtain the cell-free supernatant.

The variant polypeptides having beta-amylase activity were detected by assaying the supernatant for protein of interest expression by either SDS-PAGE or capillary electrophoresis.

### Example 4: Temperature profiles of variant beta-amylases

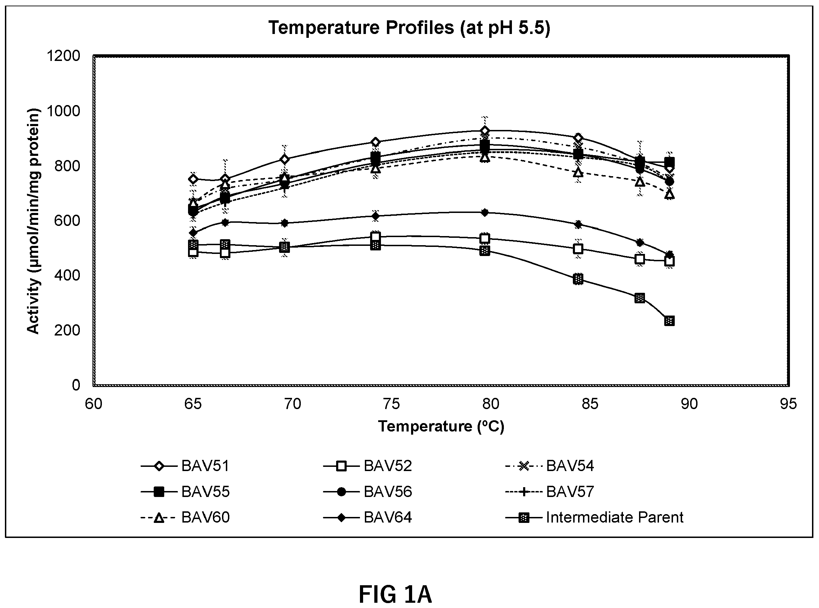

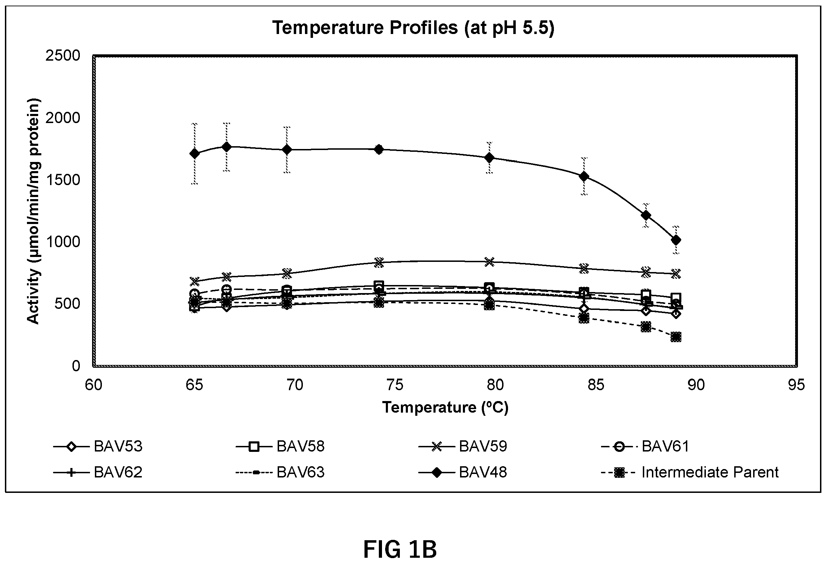

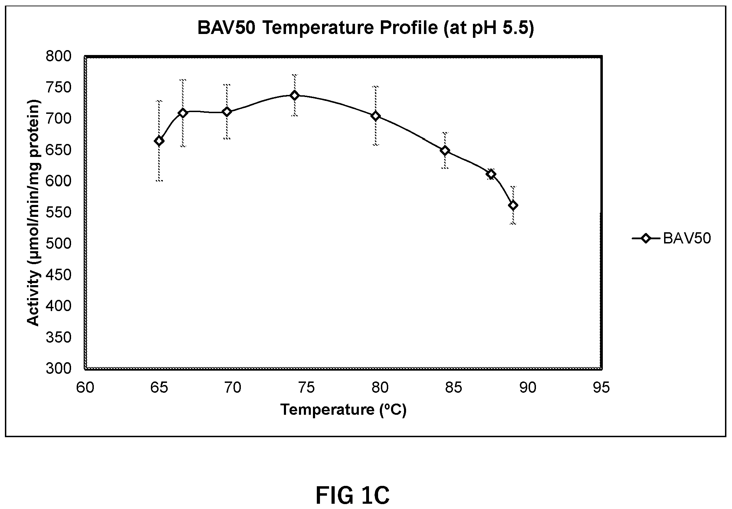

Temperature profiles of variant beta-amylases were determined using the PAHBAH assay as described in Example 1 to determine activity at temperatures of 65, 66.6, 69.6, 74.2, 79.7, 84.4, 87.5, and 89 °C at pH 5.5. The results are shown in Fig. 1A, 1B and 1C.

### Example 5: pH profiles of variant beta-amylases

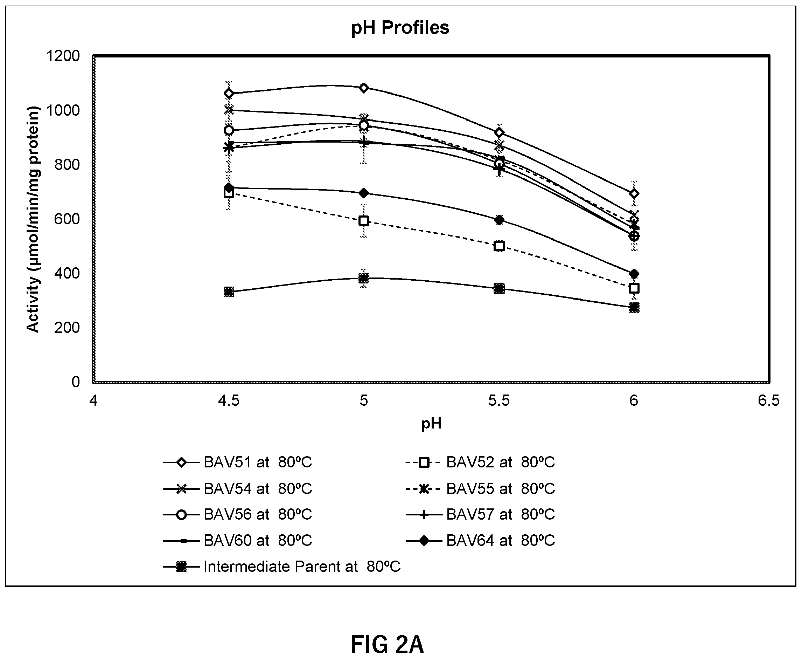

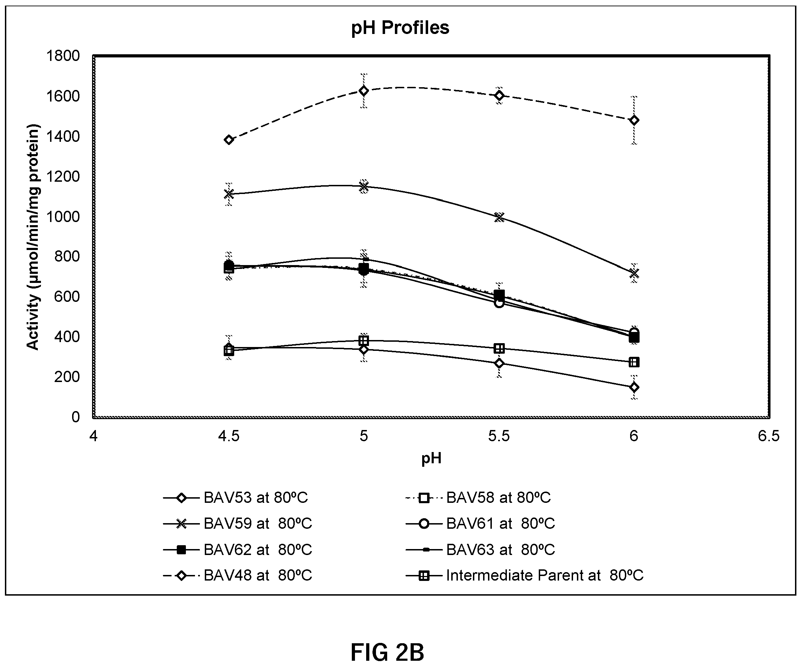

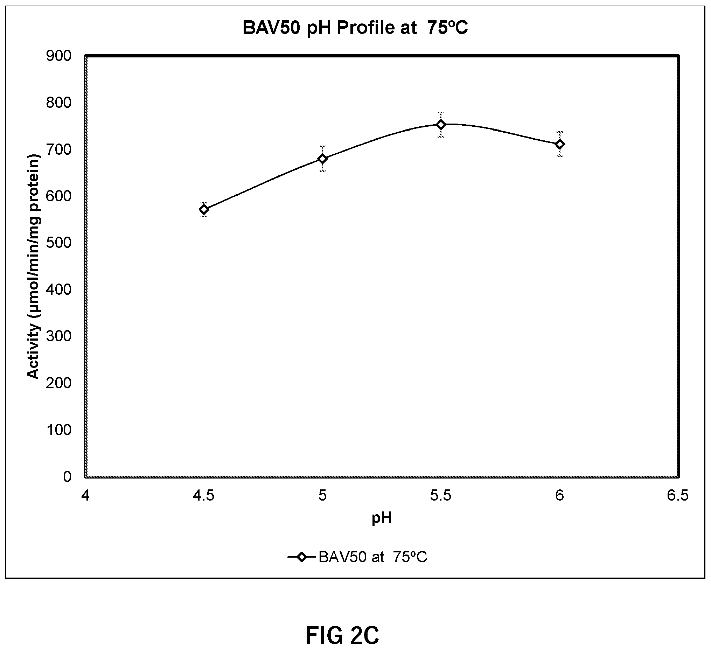

pH profiles of variant beta-amylases were determined using PAHBAH assay as described in Example 1 to determine activity at pH 4.0, 4.5, 5.0, 5.5, and 6.0 at 80°C. The results are shown in Fig. 2A, 2B and 2C.

### Example 6: Baking performance of the variant beta-amylases

The baking performance of the variant polypeptides having beta-amylase activity was tested in wheat pan bread produced in a straight process. The bread dough was prepared by mixing 1000 g of flour type 550 (Vogtmühlen Illertissen), 30 g compressed yeast, 20 g salt, 20 g sugar, 20 g margarine, 60 ppm ascorbic acid, 150 ppm Nutrilife^{®} CS 30 (fungal xylanase, cellulase, fungal alpha-amylase), 8 g Nutrisoft^{®} 55 (distilled monoglyceride) and 580 g water in a Kemper SP 15 spiral mixer for 4.5 minutes at speed 1 and 2.25 minutes at speed 2, to a final dough temperature of 28°C. After a resting of 15 minutes, the dough was divided into 500 g pieces, rounded and proofed for 15 minutes. Afterwards the dough pieces were molded, given into a baking tin and proofed for 80 minutes at 35°C at relative humidity of 85%. The proofed dough pieces were baked in a deck oven for 25 minutes at 255°C/ 240°C under lower and upper heat, with 15 seconds steam injection.

The variant polypeptide enzyme samples were added to the flour at dosages from 50 ppm to 200 ppm. The effects on the dough properties and on the final baked goods were compared to a negative control (no enzyme), and to Novamyl 3D.

The volume effect was determined by measuring the bread loafs via a laser scanner (Stable Micro Systems VolScan Profiler, VolScan 600). The negative control is defined as 0%.

Dough properties were evaluated haptically by a skilled master baker and described in comparison to the negative control.

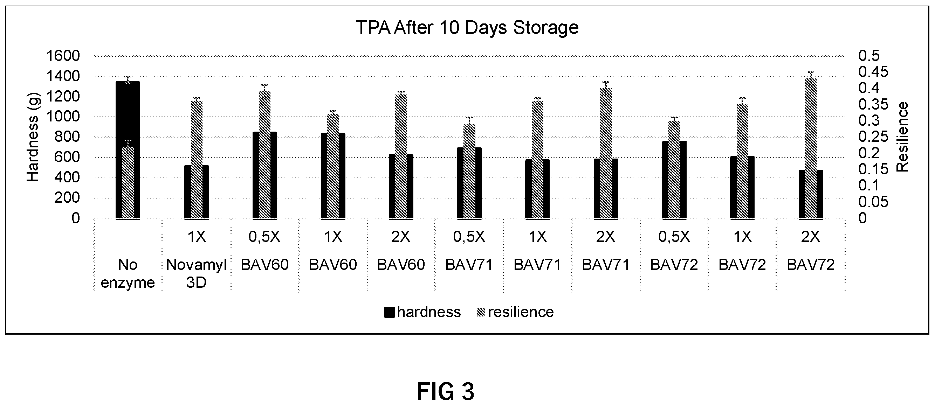

The ready baked breads were packed and sealed in a plastic bag. In addition, they partly were pasteurized for 90 minutes at 85°C. The crumb properties were determined after 10 days storage by texture profile analyses using a texture analyzer (Stable Micro Systems, TA.XTplus Texture Analyzer). 25-millimeter-thick slices were cut out of the middle of the bread loafs, prior to the measurement. The results are shown in Fig. 3.

## Claims

1. A variant polypeptide of the beta-amylase according to SEQ ID No. 1 having beta-amylase activity and comprising an amino acid sequence which is at least 80% identical to the sequence according to SEQ ID No. 1, which amino acid sequence comprises amino acid substitutions D25K, L27C, S220L, A364P, N369P, S398P and at least a first further amino acid modification at an amino acid residue position number 502 in the numbering of SEQ ID No. 1.

2. The variant polypeptide of claim 1, wherein the first further amino acid modification is an amino acid substitution, insertion, deletion, or any combination thereof.

3. The variant polypeptide of claim 2, wherein the first further amino acid modification is an amino acid substitution, and wherein the amino acid substitution is a conservative amino acid substitution.

4. The variant polypeptide of claim 2, wherein the first further amino acid modification is an amino acid substitution of T502E in the numbering of SEQ ID No. 1.

5. The variant polypeptide of claim 4, wherein the first further amino acid modification is a combination of amino acid modifications, and the combination of amino acid modifications is a combination of amino acid substitutions which is selected from the group consisting of:
(h) T494E, T502E; and
(i) N419G, T494E, T502E;
in the numbering of SEQ ID No. 1.

6. The variant polypeptide of claim 1, wherein the first further amino acid modification is a combination of amino acid modifications, and the combination of amino acid modifications is N419, T494E, T502 in the numbering of SEQ ID No. 1, and wherein the polypeptide comprises at least a second further amino acid modification.

7. The variant polypeptide of claim 6, wherein the second further amino acid modification is an amino acid substitution, insertion, deletion, or any combination thereof.

8. The variant polypeptide of claim 7, wherein the second further amino acid modification is an amino acid substitution, and wherein the amino acid substitution is a conservative amino acid substitution.

9. The variant polypeptide of claim 6, wherein the second further amino acid modification is an amino acid substitution selected from the group consisting of: P13S, A206W, V208C, Y236I, G276D, M318L, C375I, Y435P/E, N438K, and P463T or a combination thereof in the numbering of SEQ ID No. 1.

10. The variant polypeptide of claim 9, wherein the second further one amino acid modification is a combination of amino acid modifications, and the combination of amino acid modifications is a combination of amino acid substitutions which is selected from the group consisting of:
(j) A206W, V208C, G276D, M318L, C375I, Y435P, N438K, P463T;
(k) P13S, V208C, Y236I, G276D, M318L, C375I, Y435P, N438K, P463T;
(l) P13S, A206W, V208C, Y236I, G276D, M318L, C375I, Y435E, N438K, P463T;
(m) V208C, G276D, M318L, C375I, Y435P, N438K, P463T;
(n) V208C, G276D, C375I, Y435P, N438K, P463T;
(o) A206W, V208C, G276D, C375I, Y435E, N438K, P463T;
(p) A206W, V208C, G276D, C375I, Y435P, N438K, P463T;
(q) P13S, A206W, V208C, Y236I, G276D, C375I, Y435P, N438K, P463T;
(r) A206W, V208C, G276D, M318L, Y435E, N438K, P463T;
(s) A206W, V208C, G276D, M318L, C375I, Y435P, P463T;
(t) P13S, A206W, V208C, Y236I, G276D, M318L, C375I, N438K, P463T;
(u) P13S, V208C, Y236I, G276D, M318L, C375I, N438K, P463T;
(v) P13S, A206W, V208C, Y236I, G276D, M318L, C375I, N438K, P463T;
(w) P13S, A206W, V208C, Y236I, G276D, C375I, Y435P, P463T;
(x) V208C, G276D, M318L, Y435P;
(y) P13S, A206W, V208C, Y236I, G276D, M318L, P463T;
(z) A206W, V208C, G276D, M318L, P463T;
(aa) P13S, V208C, Y236I, G276D, N438K, P463T;
(bb) P13S, A206W, V208C, Y236I, G276D, M318L;
(cc) P13S, A206W, V208C, Y236I, G276D;
(dd) V208C, G276D, N419G, T494E, T502E;
(ee) C375I, N419G, T494E, T502E;
(ff) P13S, A206W, V208C, Y236I, G276D;
(gg) T90D, V91C, P269S, C375I; and
(hh) T90D, V91C, P269S, G276D, C375I;
in the numbering of SEQ ID No. 1.

11. The variant polypeptide according to any one of claims 1-10, wherein the variant polypeptide has an increased % residual activity after exposure to a temperature of 80 to 95 degrees Celsius compared to the polypeptide of SEQ ID No. 1 or 2.

12. The variant polypeptide according to claim 11, wherein the variant polypeptide has an increased % residual activity after exposure to a temperature of 86 degrees Celsius compared to the polypeptide of SEQ ID No. 1 or 2.

13. The variant polypeptide according to any one of claims 6-10, wherein the variant polypeptide has an increased % residual activity after exposure to a temperature of 90 degrees Celsius compared to the polypeptide of SEQ ID No. 1, 2, or 3.

14. The variant polypeptide according to any one of claims 6-10, wherein the variant polypeptide has an increased activity at pH 4.5-6 and a temperature of 80-85 degrees Celsius compared to the polypeptide of SEQ ID No. 1 or 2.

15. The variant polypeptide according to any one of claims 1-14 having beta-amylase activity, wherein the variant polypeptide is a fragment of the full length amino acid sequence.

16. The variant polypeptide comprising a hybrid of at least one variant polypeptide according to any one of claims 1-15, and a second polypeptide having amylase activity, wherein the hybrid has beta-amylase activity.

17. A composition comprising the variant polypeptide according to any one of claims 1-16.

18. The composition according to claim 17, further comprising a second enzyme.

19. The composition according to claim 18, wherein the second enzyme is selected from the group consisting of: an alpha-amylase, a lipase, a second beta-amylase, a G4-amylase, a xylanase, a protease, a cellulase, a glucoamylase, an oxidoreductase, a phospholipase, and a cyclodextrin glucanotransferase.

20. A method of making a variant polypeptide comprising: providing a template nucleic acid sequence encoding the polypeptide variant according to any one of claims 1-16, transforming the template nucleic acid sequence into an expression host, cultivating the expression host to produce the variant polypeptide, and purifying the variant polypeptide.

21. The method of claim 20, wherein the expression host is selected from the group consisting of: a bacterial expression system, a yeast expression system, a fungal expression system, and a synthetic expression system;
wherein the bacterial expression system is selected from an *E. coli*, a *Bacillus*, a *Pseudomonas*, and a *Streptomyces;*
wherein the yeast expression system is selected from a *Candida*, a *Pichia*, a *Saccharomyces,* a *Schizosaccharomyces;* and
wherein the fungal expression system is selected from a *Penicillium*, an *Aspergillus*, a *Fusarium*, a *Myceliopthora*, a *Rhizomucor*, a *Rhizopus*, a *Thermomyces*, and a *Trichoderma.*

22. A method of preparing a dough or a baked product prepared from the dough, the method comprising adding a variant polypeptide according to any one of claims 1-16 to the dough and eventually baking the dough.

23. The method of claim 22, wherein the baked product produced by the method exhibits lower hardness and greater resilience after 10 days of storage than a baked product produced by an otherwise identical method in which no variant polypeptide is added.

24. Use of the variant polypeptide according to any of claims 1-16 for processing starch; for cleaning or washing textiles, hard surfaces, or dishes; for making ethanol; for treating an oil well; for processing pulp or paper; for feeding an animal; for making syrup.

## Patentansprüche

1. Polypeptidvariante der Beta-Amylase gemäß SEQ ID Nr. 1, aufweisend Beta-Amylase-Aktivität und umfassend eine Aminosäuresequenz, die zu wenigstens 80 % mit der Sequenz gemäß SEQ ID Nr. 1 identisch ist, wobei die Aminosäuresequenz die Aminosäuresubstitutionen D25K, L27C, S220L, A364P, N369P, S398P und wenigstens eine erste weitere Aminosäuremodifikation an einer Aminosäurereste-Positionsnummer 502 in der Nummerierung von SEQ ID Nr. 1 umfasst.

2. Polypeptidvariante nach Anspruch 1, wobei es sich bei der ersten weiteren Aminosäuremodifikation um eine Aminosäuresubstitution, -insertion, -deletion oder eine beliebige Kombination davon handelt.

3. Polypeptidvariante nach Anspruch 2, wobei es sich bei der ersten weiteren Aminosäuremodifikation um eine Aminosäuresubstitution handelt und wobei es sich bei der Aminosäuresubstitution um eine konservative Aminosäuresubstitution handelt.

4. Polypeptidvariante nach Anspruch 2, wobei es sich bei der ersten weiteren Aminosäuremodifikation um eine Aminosäuresubstitution T502E in der Nummerierung von SEQ ID Nr. 1 handelt.

5. Polypeptidvariante nach Anspruch 4, wobei es sich bei der ersten weiteren Aminosäuremodifikation um eine Kombination von Aminosäuremodifikationen und bei der Kombination von Aminosäuremodifikationen um eine Kombination von Aminosäuresubstitutionen handelt, die aus der Gruppe bestehend aus
(h) T494E, T502E und
(i) N419G, T494E, T502E
in der Nummerierung von SEQ ID Nr. 1 ausgewählt ist.

6. Polypeptidvariante nach Anspruch 1, wobei es sich bei der ersten weiteren Aminosäuremodifikation um eine Kombination von Aminosäuremodifikationen und bei der Kombination von Aminosäuremodifikationen um N419, T494E, T502 in der Nummerierung von SEQ ID Nr. 1 handelt und wobei das Polypeptid wenigstens eine zweite weitere Aminosäuremodifikation umfasst.

7. Polypeptidvariante nach Anspruch 6, wobei es sich bei der zweiten weiteren Aminosäuremodifikation um eine Aminosäuresubstitution, -insertion, -deletion oder eine beliebige Kombination davon handelt.

8. Polypeptidvariante nach Anspruch 7, wobei es sich bei der zweiten weiteren Aminosäuremodifikation um eine Aminosäuresubstitution handelt und wobei es sich bei der Aminosäuresubstitution um eine konservative Aminosäuresubstitution handelt.

9. Polypeptidvariante nach Anspruch 6, wobei es sich bei der zweiten weiteren Aminosäuremodifikation um eine Aminosäuresubstitution, die aus der Gruppe bestehend aus P13S, A206W, V208C, Y236I, G276D, M318L, C375I, Y435P/E, N438K und P463T ausgewählt ist, oder eine Kombination davon in der Nummerierung von SEQ ID Nr. 1 handelt.

10. Polypeptidvariante nach Anspruch 9, wobei es sich bei der zweiten weiteren einen Aminosäuremodifikation um eine Kombination von Aminosäuremodifikationen und bei der Kombination von Aminosäuremodifikationen um eine Kombination von Aminosäuresubstitutionen handelt, die aus der Gruppe bestehend aus
(j) A206W, V208C, G276D, M318L, C375I, Y435P, N438K, P463T;
(k) P13S, V208C, Y236I, G276D, M318L, C375I, Y435P, N438K, P463T;
(l) P13S, A206W, V208C, Y236I, G276D, M318L, C375I, Y435E, N438K, P463T;
(m) V208C, G276D, M318L, C375I, Y435P, N438K, P463T;
(n) V208C, G276D, C375I, Y435P, N438K, P463T;
(o) A206W, V208C, G276D, C375I, Y435E, N438K, P463T;
(p) A206W, V208C, G276D, C375I, Y435P, N438K, P463T;
(q) P13S, A206W, V208C, Y236I, G276D, C375I, Y435P, N438K, P463T;
(r) A206W, V208C, G276D, M318L, Y435E, N438K, P463T;
(s) A206W, V208C, G276D, M318L, C375I, Y435P, P463T;
(t) P13S, A206W, V208C, Y236I, G276D, M318L, C375I, N438K, P463T;
(u) P13S, V208C, Y236I, G276D, M318L, C375I, N438K, P463T;
(v) P13S, A206W, V208C, Y236I, G276D, M318L, C375I, N438K, P463T;
(w) P13S, A206W, V208C, Y236I, G276D, C375I, Y435P, P463T;
(x) V208C, G276D, M318L, Y435P;
(y) P13S, A206W, V208C, Y236I, G276D, M318L, P463T;
(z) A206W, V208C, G276D, M318L, P463T;
(aa) P13S, V208C, Y236I, G276D, N438K, P463T;
(bb) P13S, A206W, V208C, Y236I, G276D, M318L;
(cc) P13S, A206W, V208C, Y236I, G276D;
(dd) V208C, G276D, N419G, T494E, T502E;
(ee) C375I, N419G, T494E, T502E;
(ff) P13S, A206W, V208C, Y236I, G276D;
(gg) T90D, V91C, P269S, C375I und
(hh) T90D, V91C, P269S, G276D, C375I
in der Nummerierung von SEQ ID Nr. 1 ausgewählt ist.

11. Polypeptidvariante gemäß einem der Ansprüche 1-10, wobei die Polypeptidvariante, nachdem sie einer Temperatur von 80 bis 95 Grad Celsius ausgesetzt wurde, gegenüber dem Polypeptid unter SEQ ID Nr. 1 oder 2 eine erhöhte %-Restaktivität aufweist.

12. Polypeptidvariante gemäß Anspruch 11, wobei die Polypeptidvariante, nachdem sie einer Temperatur von 86 Grad Celsius ausgesetzt wurde, gegenüber dem Polypeptid unter SEQ ID Nr. 1 oder 2 eine erhöhte %-Restaktivität aufweist.

13. Polypeptidvariante gemäß einem der Ansprüche 6-10, wobei die Polypeptidvariante, nachdem sie einer Temperatur von 90 Grad Celsius ausgesetzt wurde, gegenüber dem Polypeptid unter SEQ ID Nr. 1, 2 oder 3 eine erhöhte %-Restaktivität aufweist.

14. Polypeptidvariante gemäß einem der Ansprüche 6-10, wobei die Polypeptidvariante eine erhöhte Aktivität bei pH 4,5-6 und einer Temperatur von 80-85 Grad Celsius gegenüber dem Polypeptid unter SEQ ID Nr. 1 oder 2 aufweist.

15. Polypeptidvariante gemäß einem der Ansprüche 1-14 mit Beta-Amylase-Aktivität, wobei es sich bei der Polypeptidvariante um ein Fragment der Volllängen-Aminosäuresequenz handelt.

16. Polypeptidvariante, umfassend ein Hybrid wenigstens einer Polypeptidvariante gemäß einem der Ansprüche 1-15 und eines zweiten Polypeptids mit Amylase-Aktivität, wobei das Hybrid Beta-Amylase-Aktivität aufweist.

17. Zusammensetzung, umfassend die Polypeptidvariante gemäß einem der Ansprüche 1-16.

18. Zusammensetzung gemäß Anspruch 17, ferner umfassend ein zweites Enzym.

19. Zusammensetzung gemäß Anspruch 18, wobei das zweite Enzym aus der Gruppe bestehend aus einer Alpha-Amylase, einer Lipase, einer zweiten Beta-Amylase, einer G4-Amylase, einer Xylanase, einer Protease, einer Cellulase, einer Glucoamylase, einer Oxidoreduktase, einer Phospholipase und einer Cyclodextrin-Glucanotransferase ausgewählt ist.

20. Verfahren zur Herstellung einer Polypeptidvariante, umfassend: Bereitstellen einer Matrizen-Nukleinsäuresequenz, die die Polypeptidvariante gemäß einem der Ansprüche 1-16 codiert, Transformieren der Matrizen-Nukleinsäuresequenz in einen Expressionswirt, Kultivieren des Expressionswirts unter Erhalt der Polypeptidvariante und Aufreinigen der Polypeptidvariante.

21. Verfahren nach Anspruch 20, wobei der Expressionswirt aus der Gruppe bestehend aus einem bakteriellen Expressionssystem, einem Hefe-Expressionssystem, einem Pilz-Expressionssystem und einem synthetischen Expressionssystem ausgewählt ist;
wobei das bakterielle Expressionssystem aus einem *E. coli*, einem *Bacillus*, *einem Pseudomonas* und einem *Streptomyces* ausgewählt ist;
wobei das Hefe-Expressionssystem aus einer *Candida*, einer *Pichia*, einer *Saccharomyces*, einer *Schizosaccharomyces* ausgewählt ist; und
wobei das Pilz-Expressionssystem aus einem *Penicillium*, einem *Aspergillus*, einem *Fusarium*, einem *Myceliopthora*, einem *Rhizomucor*, einem *Rhizopus*, einem *Thermomyces* und einem *Trichoderma* ausgewählt ist.

22. Verfahren zur Herstellung eines Teigs oder eines aus dem Teig hergestellten Backprodukts, wobei das Verfahren Zugeben einer Polypeptidvariante gemäß einem der Ansprüche 1-16 zum Teig und schließlich Backen des Teigs umfasst.

23. Verfahren nach Anspruch 22, wobei das mit dem Verfahren erzeugte Backprodukt nach 10 Tagen Lagerung eine geringere Härte und größere Nachgiebigkeit als ein mit einem ansonsten identischen Verfahren, bei dem keine Zugabe einer Polypeptidvariante erfolgt, erzeugtes Backprodukt zeigt.

24. Verwendung der Polypeptidvariante gemäß einem der Ansprüche 1-16 zum Verarbeiten von Stärke; zum Reinigen oder Waschen von Textilien, harten Oberflächen oder Geschirr; zum Herstellen von Ethanol; zum Behandeln einer Ölquelle; zum Verarbeiten von Zellstoff oder Papier; zur Fütterung eines Tiers; zum Herstellen von Sirup.

## Revendications

1. Polypeptide variant de la bêta-amylase selon la SEQ ID N° 1 ayant une activité de bêta-amylase et comprenant une séquence d'acides aminés qui est au moins 80 % identique à la séquence selon SEQ ID N° 1, laquelle séquence d'acides aminés comprend les substitutions d'acides aminés D25K, L27C, S220L, A364P, N369P, S398P et au moins une première autre modification d'acide aminé au niveau d'une position de résidu d'acide aminé numéro 502 dans la numérotation de la SEQ ID N° 1.

2. Polypeptide variant selon la revendication 1, dans lequel la première autre modification d'acide aminé est une substitution, insertion, délétion d'acide aminé ou toute combinaison de celles-ci.

3. Polypeptide variant selon la revendication 2, dans lequel la première autre modification d'acide aminé est une substitution d'acide aminé, et dans lequel la substitution d'acide aminé est une substitution d'acide aminé conservatrice.

4. Polypeptide variant selon la revendication 2, dans lequel la première autre modification d'acide aminé est une substitution d'acide aminé de T502E dans la numérotation de la SEQ ID N° 1.

5. Polypeptide variant selon la revendication 4, dans lequel la première autre modification d'acide aminé est une combinaison de modifications d'acides aminés, et la combinaison de modifications d'acides aminés est une combinaison de substitutions d'acides aminés qui est choisie dans le groupe constitué par :
(h) T494E, T502E ; et
(i) N419G, T494E, T502E ;
dans la numérotation de la SEQ ID N° 1.

6. Polypeptide variant selon la revendication 1, dans lequel la première autre modification d'acide aminé est une combinaison de modifications d'acides aminés, et la combinaison de modifications d'acides aminés est N419, T494E, T502 dans la numérotation de la SEQ ID N° 1, et dans lequel le polypeptide comprend au moins une deuxième autre modification d'acides aminés.

7. Polypeptide variant selon la revendication 6, dans lequel la deuxième autre modification d'acide aminé est une substitution, insertion, délétion d'acide aminé ou toute combinaison de celles-ci.

8. Polypeptide variant selon la revendication 7, dans lequel la deuxième autre modification d'acide aminé est une substitution d'acide aminé, et dans lequel la substitution d'acide aminé est une substitution d'acide aminé conservatrice.

9. Polypeptide variant selon la revendication 6, dans lequel la deuxième autre modification d'acide aminé est une substitution d'acide aminé choisie dans le groupe constitué par : P13S, A206W, V208C, Y236I, G276D, M318L, C375I, Y435P/E, N438K et P463T ou une combinaison de celles-ci dans la numérotation de la SEQ ID N° 1.

10. Polypeptide variant selon la revendication 9, dans lequel la deuxième autre modification d'acide aminé est une combinaison de modifications d'acides aminés, et la combinaison de modifications d'acides aminés est une combinaison de substitutions d'acides aminés qui est choisie dans le groupe constitué par :
(j) A206W, V208C, G276D, M318L, C375I, Y435P, N438K, P463T ;
(k) P13S, V208C, Y236I, G276D, M318L, C375I, Y435P, N438K, P463T ;
(l) P13S, A206W, V208C, Y236I, G276D, M318L, C375I, Y435E, N438K, P463T ;
(m) V208C, G276D, M318L, C375I, Y435P, N438K, P463T ;
(n) V208C, G276D, C375I, Y435P, N438K, P463T ;
(o) A206W, V208C, G276D, C375I, Y435E, N438K, P463T ;
(p) A206W, V208C, G276D, C375I, Y435P, N438K, P463T ;
(q) P13S, A206W, V208C, Y236I, G276D, C375I, Y435P, N438K, P463T ;
(r) A206W, V208C, G276D, M318L, Y435E, N438K, P463T ;
(s) A206W, V208C, G276D, M318L, C375I, Y435P, P463T ;
(t) P13S, A206W, V208C, Y236I, G276D, M318L, C375I, N438K, P463T ;
(u) P13S, V208C, Y236I, G276D, M318L, C375I, N438K, P463T ;
(v) P13S, A206W, V208C, Y236I, G276D, M318L, C375I, N438K, P463T ;
(w) P13S, A206W, V208C, Y236I, G276D, C375I, Y435P, P463T ;
(x) V208C, G276D, M318L, Y435P ;
(y) P13S, A206W, V208C, Y236I, G276D, M318L, P463T ;
(z) A206W, V208C, G276D, M318L, P463T ;
(aa) P13S, V208C, Y236I, G276D, N438K, P463T ;
(bb) P13S, A206W, V208C, Y236I, G276D, M318L ;
(cc) P13S, A206W, V208C, Y236I, G276D ;
(dd) V208C, G276D, N419G, T494E, T502E ;
(ee) C375I, N419G, T494E, T502E ;
(ff) P13S, A206W, V208C, Y236I, G276D ;
(gg) T90D, V91C, P269S, C375I ; et
(hh) T90D, V91C, P269S, G276D, C375I ;
dans la numérotation de la SEQ ID N° 1.

11. Polypeptide variant selon l'une quelconque des revendications 1 à 10, dans lequel le polypeptide variant a un % d'activité résiduelle augmenté après exposition à une température de 80 à 95 degrés Celsius par rapport au polypeptide de la SEQ ID N° 1 ou 2.

12. Polypeptide variant selon la revendication 11, dans lequel le polypeptide variant a un % d'activité résiduelle augmenté après exposition à une température de 86 degrés Celsius par rapport au polypeptide de la SEQ ID N° 1 ou 2.

13. Polypeptide variant selon l'une quelconque des revendications 6 à 10, dans lequel le polypeptide variant a un % d'activité résiduelle augmenté après exposition à une température de 90 degrés Celsius par rapport au polypeptide de la SEQ ID N° 1, 2 ou 3.

14. Polypeptide variant selon l'une quelconque des revendications 6 à 10, dans lequel le polypeptide variant a une activité augmentée à pH 4,5 à 6 et une température de 80 à 85 degrés Celsius par rapport au polypeptide de la SEQ ID N° 1 ou 2.

15. Polypeptide variant selon l'une quelconque des revendications 1-14 ayant une activité de bêta-amylase, dans lequel le polypeptide variant est un fragment de la séquence d'acides aminés de pleine longueur.

16. Polypeptide variant comprenant un hybride d'au moins un polypeptide variant selon l'une quelconque des revendications 1 à 15, et un deuxième polypeptide ayant une activité d'amylase, dans lequel l'hybride a une activité de bêta-amylase.

17. Composition comprenant le polypeptide variant selon l'une quelconque des revendications 1 à 16.

18. Composition selon la revendication 17, comprenant en outre une deuxième enzyme.

19. Composition selon la revendication 18, dans laquelle la deuxième enzyme est choisie dans le groupe constitué par : une alpha-amylase, une lipase, une seconde bêta-amylase, une G4-amylase, une xylanase, une protéase, une cellulase, une glucoamylase, une oxydoréductase, une phospholipase, et une cyclodextrine glucanotransférase.

20. Procédé de préparation d'un polypeptide variant comprenant : la fourniture d'une séquence d'acide nucléique de matrice codant pour le variant polypeptidique selon l'une quelconque des revendications 1 à 16, la transformation de la séquence d'acide nucléique de matrice en un hôte d'expression, la culture de l'hôte d'expression pour produire le polypeptide variant, et la purification du polypeptide variant.

21. Procédé selon la revendication 20, dans lequel l'hôte d'expression est choisi dans le groupe constitué par : un système d'expression bactérienne, un système d'expression de levure, un système d'expression fongique et un système d'expression synthétique ;
dans lequel le système d'expression bactérienne est choisi parmi *E. coli*, un *Bacillus*, *Pseudomonas* et un *Streptomyces* ;
dans lequel le système d'expression de levure est choisi parmi un *Candida*, un *Pichia*, un *Saccharomyces*, un *Schizosaccharomyces* ; et
dans lequel le système d'expression fongique est choisi parmi un *Pénicillium*, un *Aspergillus,* un *Fusarium*, un *Myceliopthora*, un *Rhizomucor*, un Rhizopus, un *Thermomyces* et un *Trichoderma.*

22. Procédé de préparation d'une pâte ou d'un produit cuit préparé à partir de la pâte, le procédé comprenant l'ajout d'un polypeptide variant selon l'une quelconque des revendications 1-16 à la pâte et éventuellement la cuisson de la pâte.

23. Procédé selon la revendication 22, dans lequel le produit cuit produit par le procédé présente une dureté inférieure et une résilience supérieure après 10 jours de stockage par rapport à un produit cuit produit par un procédé autrement identique dans lequel aucun polypeptide variant n'est ajouté.

24. Utilisation du polypeptide variant selon l'une quelconque des revendications 1-16 pour traiter de l'amidon ; pour nettoyer ou laver des textiles, des surfaces dures ou de la vaisselle ; pour fabriquer de l'éthanol ; pour traiter un puits de pétrole ; pour traiter de la pâte ou du papier ; pour nourrir un animal ; pour fabriquer un sirop.
